Europäisches Patentamt

European Patent Office

Office européen des brevets

(19)

(11) Publication number: **0 125 759 B1**

# EUROPEAN PATENT SPECIFICATION

(12)

(45) Date of publication of patent specification: **25.09.91**   (51) Int. Cl.⁵: **A61K 9/06**, A61K 47/00

(21) Application number: **84301806.0**

(22) Date of filing: **16.03.84**

(54) Topical composition of antibiotics for application to the mucosa.

(30) Priority: **17.03.83 FR 8304378**

(43) Date of publication of application:
**21.11.84 Bulletin 84/47**

(45) Publication of the grant of the patent:
**25.09.91 Bulletin 91/39**

(84) Designated Contracting States:
**AT BE CH DE FR GB IT LI NL SE**

(56) References cited:
**DE-A- 3 200 766**
**DE-C- 959 219**
**US-A- 4 318 746**

(73) Proprietor: **WARNER LAMBERT HOLLAND B.V.**
**Oderweg 1**
**NL-1043 AG Amsterdam(NL)**

(72) Inventor: **Piffeteau, Pierre**
**13 rue Linne**
**F-75005 Paris(FR)**

(74) Representative: **Hirsch, Marc-Roger et al**
**Cabinet Hirsch 34 rue de Bassano**
**F-75008 Paris(FR)**

Rank Xerox (UK) Business Services

## Description

The invention relates to a composition for topical application to the skin and the mucosa, particularly to the oral and genital mucosae, for the treatment of infections, especially fungal infections such as candidosis.

US P 4 318 746 discloses the use of a gel comprising
- a first polymer that dissolves, hydrates or disperses in hot water and forms a rigid gel on cooling;
- a second polymer insoluble in hot water that dissolves or hydrates on cooling and is compatible with said first polymer;
- water, the combination of said polymers being made in hot water;

and, in fact, according to said USP 4 318 746 :
"high temperature stability at about 80 °C is believed to be caused by the use of both a hot water soluble polymer and a hot water insoluble polymer".

Candidosis is a condition affecting human perspiring skin and mucosa, especially that of the oral cavity and of the vagina, which is due to an infection of the yeast Candida albicans. This condition is also known colloquially as "thrush" or "lily of the valley" (muguet), and can be treated by topical application of antifungal agents such as Nystatin. It is however apparent that such an infection following normal dosage of this fungicidal antibiotic can be slow to clear because of the difficulty in applying it to, and maintaining it in contact with the infected mucosa for a sufficient period of time. In the mouth, for example, normal salivary flow is sufficient to dislodge and wash away from the infected site a preparation containing Nystatin shortly after application, so that Nystatin has insufficient time in contact with the infecting yeast to reduce and eliminate the infection. Likewise, in the vulvo-vaginal region, natural secretions exacerbated by candidosis can rapidly dislodge topically applied Nystatin before it can take effect, so that treatment of the disease can be slow or ineffective.

In order to achieve a more effective and rapid response to treatment, it would accordingly be necessary to apply the fungicidal antibiotic topically to the infected mucosa in an unacceptably high dose. It is therefore with the provision of a special vehicle to fix the anti-fungal agent to the affected mucosa for a sufficient time to effect a remission of the disease that this invention is concerned.

We have accordingly now found that by including a special degraded hydrocolloid in the formulation containing Nystatin, it is possible to increase dramatically the adhesion of Nystatin to skin, particularly to the mucosa, for example Candida albicans infected mucosa, to such an extent that rapid regression of the infection can be achieved. It is also apparent that the special degraded hydrocolloid is also effective in fixing other antibiotics to the mucosa for a period of time which is longer than expected, in order to achieve enhanced regression of mucosa-borne fungal infections.

It is believed that since degraded hydrocolloid is a negatively charged polymer based on sulphated polygalactosides having a relatively low molecular weight, it is capable of forming complexes with positively charged polymers such as proteins, and hence it is particularly suited to reacting with the protein-rich cells of Candida albicans which abound at the site of the candidosis infection. The special degraded hydrocolloid accordingly can function as a fixative agent for a fungicidal antibiotic by improving the delivery and increasing the duration of contact between the site of infection and the applied antibiotic, with consequent more rapid remission of the disease.

The invention therefore provides a pharmacologically acceptable aqueous composition for topical application to the skin and the mucosa, which comprises:

(a) a pharmacologically effective amount Of from 0.3 to 30% by weight of an antibiotic; and

(b) from 1 to 50% by weight of a hydrocolloid polymer comprising sulphated polygalactoside, and having a molecular weight of from 10,000 to 20,000 which is capable of forming a film of gel as a fixative agent for the antibiotic on contact with proteins.

The aqueous pharmacologically acceptable composition according to the invention will comprise an antibiotic which is suitable for topical application to skin, and in particular to the mucosa, particularly for the treatment of infections due to yeasts or other fungi.

The antibiotic can be chosen from any of the following examples of pharmacologically acceptable antibiotics.

Nystatin

Miconazole nitrate

Lauryl oxypropyl β-amino butyric acid

Amphotericin B

Undecylenic acid

Chloroquinaldol

Econazole nitrate

Natamycin
Cloprothiazole
Clotrimazole
Tolnaftate
Lucensomycin
Cyclins, such as Tetracyclins
Macrolides, such as Erythromycin.

It is also possible to use a mixture of two or more of the above antibiotics, as well as other antibiotics not listed above.

The amount of antibiotic to be employed in the composition will depend on its activity and the nature of the infection of the mucosa to which it is applied. The expression "a pharmacologically effective amount" is accordingly such an amount as is considered by the manufacturer or supplier of the antibiotic or the physician prescribing its use to be sufficient for the treatment of an infection.

By way of example, it can be stated that for the preferred fungicidal antibiotic, Nystatin, which is normally supplied in a form having a minimum activity of 3,000,000 U per g, calculated with reference to the dried substance, the composition can conveniently contain from 10,000 to 1,000,000 U, preferably from 20,000 to 500,000 U Nystatin per g of the composition. This is equivalent to a concentration of Nystatin in the composition of from about 0.3 to about 30%, preferably about 0.6 to about 15% by weight, the Nystatin having an activity which is at least the minimum of that defined by the WHO standard.

It can be stated generally that these quantities are appropriate for a wide range of antibiotics, including those specifically referred to above.

The aqueous pharmacologically acceptable composition according to the invention will also comprise, as a fixative agent for the antibiotic, a negatively charged hydrocolloid polymer comprising sulphated polygalactoside, which is capable of forming a gel, the polymer having a molecular weight of from 10,000 to 20,000.

This polymer is particularly characterised by its capability of interacting with proteins, such as that to be found in abundance where infective yeast or fungal cells or mycelia proliferate on the mucosa, to form an adhesive union therewith. By this means, it is believed that the duration of exposure of the cells or mycelia to an antibiotic carried by the polymer is greatly increased, thus effecting a rapid remission of the infection.

Examples of the hydrocolloid polymer having these characteristics are degraded lambda - carrageenan, degraded kappa - carrageenan and degraded iota - carrageenan, of which the degraded kappa and iota forms are preferred.

The most preferred form of the hydrocolloid polymer is a sulphated form of mixed degraded carrageenans of which degraded kappa - carrageenan predominates, obtained by extraction of the red alga Chondrus crispus followed by hydrolysis to reduce the molecular weight to a value of from 10,000 to 20,000. An alternative and equally preferred hydrocolloid polymer is a highly sulphated form of degraded iota-carrageenan obtained by extraction of the red alga, Eucheuma spinosum, followed by hydrolysis to reduce the molecular weight to a value within the same range.

The amount of the fixative agent to be employed in the composition will generally be from 1 to 50%, preferably from 5 to 20% by weight of the composition.

It is apparent that compositions containing less than 1% by weight of the fixative agent are unlikely to exhibit enhanced delivery and substantivity of the antibiotic to the mucosae, whereas, compositions containing more than 50% by weight of the fixative agent are usually unmanageably viscous and difficult to apply topically and are therefore unsatisfactory to use from the consumer's point of view. Furthermore, enhancement of delivery and substantivity of the antibiotic is unlikely to be further enhanced by employing in the composition more than 50% by weight of the fixative agent.

It is also apparent that compositions containing an undegraded, or a partially degraded hydrocolloid polymer, having a molecular weight in excess of 20,000, exhibit poor film forming ability and fixative properties to the mucosa, such that the residence time during which the antibiotic remains in contact with the infected area is too short to effect rapid remission of the condition. Similarly, overdegraded hydrocolloid polymers, having a molecular weight of less than 10,000, lack the necessary fixative properties which are a necessary feature of the invention.

The hydrocolloid polymer having a molecular weight of from 10,000 to 20,000 can be prepared for example from kappa-, lambda- or ioto-carrageenan, or a mixture thereof as is present in the preferred red algae Chondrus crispus, Eucheuma spinosum, or in other species of algae such as Iridea sp, Eucheuma cottonii and Gigartina acicularis, by a process which includes the following steps:

(i) wash the algae and macerate in hot water containing calcium hydroxide to extract a gum which is largely undernatured carrageenan;

3

(ii) filter the hot extract to remove insolubles and to obtain a thin syrup containing undegraded carrageenan in solution;

(iii) precipitate the undegraded carrageenan with isopropyl alcohol;

(iv) separate the precipitate and wash with alcohol and finally dry the precipitate to obtain dried undegraded carrageenan;

(v) resuspend the purified undegraded carrageenan in isopropyl alcohol and effect partial depolymerisation by the addition of hydrogen peroxide.

The product finally obtained is degraded carrageenan which contains a substantial proportion of hydrocolloidal polymer having a molecular weight of from 10,000 to 20,000.

The viscosity of this material as a 5% by weight solution in water at 20°C is not greater than 10 mPas (cps), as measured in the Brookfield viscometer fitted with a No.1 needle at 60 rotations a minute.

The composition according to the invention will also comprise from 10 to 99%, preferably 20 to 95% and most preferably 50 to 93% by weight of water.

The aqueous pharmacologically acceptable composition according to the invention can also optionally comprise one or more excipients in addition to water to enable the composition to be more readily formulated, dispersed and applied to the mucosa.

Examples of excipients include pharmacologically inert thickeners, diluents, solvents, emollients, flavouring materials, preservatives, antioxidants, emulsifiers and surfactants.

A particularly preferred excipient for use as a thickener and gelling agent when the composition takes the form of a gel is a chemically modified cellulose derivative such as hydroxymethyl cellulose.

The amount of excipient that can be employed in the composition will generally form the balance, if any, remaining after taking account of the essential ingredients as defined herein. Accordingly, the excipient or excipients can form from 0.1 to 98.7%, preferably from 1 to 50% and most preferably from 1 to 20% by weight of the composition.

The invention also provides a process for the preparation of an aqueous pharmacologically acceptable composition for topical application to the mucosa, which comprises the step of mixing together an antibiotic, a degraded hydrocolloid comprising sulphated polygalactoside having a molecular weight of from 10,000 to 20,000 as herein defined, as a fixative agent for the antibiotic, and water, to provide an aqueous composition in which the antibiotic is present in a pharmacologically effective amount, the fixative agent forming from 1 to 50% by weight of the composition.

The composition according to the invention can be prepared in the form of a lotion or gel. It will normally be packed in a closed container such as a tube or lidded jar and can conveniently be applied to the skin, and especially to the mucosa with a suitable applicator in a suitable amount such as a unit dose as directed by the physician.

The invention also provides a mixture of Nystatin and polygeenan for the topical treatment of candidosis.

The invention is further illustrated by the following Examples.

Example 1

This Example illustrates a fungicidal gel composition containing Nystatin as the antibiotic. A gel composition was prepared from the following ingredients:

| Fungicidal antibiotic | % w/w |
|---|---|
| Nystatin* | 3.3 |

**Fixative agent**

degraded kappa-carrageenan
(mol. wt. 10,000-20,000) derived
from <u>Chondrus crispus</u>

**Excipients**

| preservatives | 0.15 |
|---|---|
| hydroxyethyl cellulose (thickener) | 1.5 |
| buffer | 0.7 |
| antioxidants | 0.02 |
| emulsifier | 0.08 |
| Water (purified) | to 100 |

*having an activity of 3,000,000 U Per g.

This gel can be applied to the mucosa, preferably with a suitable tubular applicator fitted to a compressible aluminium foil tube. Topical application in this manner to the oral or genital mucosae can provide a rapid and effective regression of a mucosa-borne infection such as candidosis.

Example 2

This Example illustrates a fungicidal gel composition containing Amphotericin B as the antibiotic. A gel composition was prepared from the following ingredients:

| Fungicidal antibiotic | % w/w |
|---|---|
| Amphotericin B | 3 |

| Fixative agent | |
|---|---|
| degraded iota-carrageenan (mol. wt. 10,000-20,000) derived from _Eucheuma spinosum_ | 10 |

| Excipients | |
|---|---|
| hydroxyethyl cellulose (thickener) | 1.5 |
| preservatives | 0.11 |
| antioxidants | 0.02 |
| emulsifier | 0.08 |
| buffer | 0.5 |
| Water (purified) | to 100 |

Example 3

This Example illustrates a fungicidal gel composition containing Natamycin (Pimaricin) as the antibiotic. A gel composition was accordingly prepared using the same ingredients as employed in Example 2, except that the Amphotericin B was replaced with 2g Natamycin.

Example 4

This Example illustrates a fungicidal gel composition containing Lucensomycin as the antibiotic. A gel composition was accordingly prepared using the same ingredients as employed in Example 2, except that the Amphotericin B was replaced with 2g Lucensomycin.

Example 5

This Example illustrates a fungicidal gel composition containing Econazole nitrate as the antibiotic. A gel composition was accordingly prepared using the same ingredients as employed in Example 2, except that the Amphotericin B was replaced with Econazole nitrate.

Example 6

This Example illustrates a fungicidal gel composition containing Tetracyclin base as the antibiotic. A gel composition was prepared from the following ingredients:

|                                                                                         | % w/w   |
| --------------------------------------------------------------------------------------- | ------- |
| **Fungicidal antibiotic**                                                               |         |
| Tetracyclin base                                                                        | 1.0     |
|                                                                                         |         |
| **Fixative agent**                                                                      |         |
| degraded iota-carrageenan (mol. wt. 10,000 to 20,000) derived from _Eucheuma spinosum_  | 10      |
|                                                                                         |         |
| **Excipients**                                                                          |         |
| hydroxyethyl cellulose (thickener)                                                      | 1.5     |
| buffer salts                                                                            | 0.7     |
| Water (purified)                                                                        | to 100  |

Example 7

This Example illustrates a fungicidal gel composition containing Erythromycin base as the antibiotic. A gel composition was prepared from the following ingredients.

|                                                                                         | % w/w   |
| --------------------------------------------------------------------------------------- | ------- |
| **Fungicidal antibiotic**                                                               |         |
| Erythromycin base                                                                       | 4       |
|                                                                                         |         |
| **Fixative agent**                                                                      |         |
| degraded kappa-carrageenan (mol. wt. 10,000 to 20,000) derived from _Chondrus crispus_  | 10      |
|                                                                                         |         |
| **Excipients**                                                                          |         |
| hydroxyethyl cellulose (thickener)                                                      | 1.5     |
| antioxidants                                                                            | 0.02    |
| emulsifier                                                                              | 0.08    |
| buffer salts                                                                            | 1.4     |
| Water (purified)                                                                        | to 100  |

Example 8

This Example illustrates a fungicidal gel composition containing Chlorquinaldol as the antibiotic. A gel composition was prepared from the following ingredients:

| | **% w/w** |
|---|---|
| <u>**Fungicidal antibiotic**</u> | |
| Chlorquinaldol | 1 |
| <u>**Fixative agent**</u> | |
| degraded kappa-carrageenan (mol. wt 10,000 to 20,000) derived from <u>Chondrus crispus</u> | 10 |
| <u>**Excipients**</u> | |
| hydroxy ethyl cellulose (thickener) | 1.5 |
| buffer salts | 0.7 |
| Water | to 100 |

Clinical Evidence

Human subjects suffering from candidosis have been treated successfully by topical application of a composition containing Nystatin according to the invention.

Three types of conditions were treated; these were external dermatological, vaginal and oral candidosis. In each case a Nystatin containing composition as described in Example 1 was employed, the application of the composition being carried out under strictly controlled conditions by an experienced dermatologist, gynaecologist or paediatrician, or when appropriate by the subject under supervised conditions. The results of the respective treatments were scrutinised and reported by the respective clinician.

1. External Dermatological Candidosis

14 subjects (male and female) suffering from superficial Candida albicans infection (acute candidosis) were selected for a clinical trial using the composition of Example 1.

These subjects presented infected skin folds and infections of the perianal and perigenital regions, including intertrigo, onchychitis, koilonychia, perleche, balanitis and anal mycosis.

Each subject received two applications per day to the affected area, of the composition of Example 1, for a minimum of 15 days. Some treatments were extended to more than 21 days.

At the termination of treatment, the condition of each subject was assessed by the dermatologist and in every case, there was no trace of Candida albicans infection.

2. Vulvovaginal Candidosis

10 female subjects suffering from Candida albicans infection of the vulvo-vaginal region (acute vaginal candidosis) were similarly selected for a clinical trial using the composition of Example 1.

Each subject received two applications per day, one in the morning and one at night, to the affected area, and their condition was again assessed by the dermatologist after 15 days.

In each case, candidosis had been completely eliminated.

3. Oral Candidosis

Three infants each suffering from Candida albicans infection of the mouth (acute oral candidosis), were similarly selected for a clinical trial using the Nystatin-containing composition of Example 1.

Regular application of this composition to the affected oral mucosa 1.5 hours after feeding resulted in a rapid and complete remission of the disease.

It can be concluded from these clinical data that the composition according to the invention as tested proved to be extremely effective in rapidly irradicating acute candidosis.

## Claims

1. Composition for topical application to the skin and the mucosa, particularly to the oral and genital mucosae, for the treatment of infections, comprising

   a) a pharmacologically effective amount of from 0.3 to 3% by weight of an antibiotic, and

   b) as a fixative agent for the antibiotic, from 1 to 50% by weight of a hydrocolloid polymer comprising sulphated polygalactoside and having a molecular weight of from 10,000 to 20,000 which is capable of forming a gel.

2. A composition according to claim 1, characterised in that the antibiotic is chosen from:

   Nystatin
   Miconazole nitrate
   Lauryl oxpropyl $\beta$-amino butyric acid
   Amphotericin B
   Undecylenic acid
   Chlorquinaldol
   Econazole nitrate
   Natamycin
   Cloprothiazole
   Clotrimazole
   Tolnaftate
   Lucensomycin
   Tetracyclin
   Erythromycin

3. A composition according to claim 1 or 2, characterised in that the fixative agent is chosen from degraded lambda-carrageenan, degraded kappa-carrageenan degraded iota-carrageenan, or mixtures thereof, each having a molecular weight of from 10,000 to 20,000.

4. A composition according to claim 1 or 2, characterised in that the fixative agent is derived from the red alga Chondrus crispus.

5. A composition according to claim 1 or 2, characterised in that the fixative agent is derived from the red alga Eucheuma spinosum.

6. A composition according to any preceding claim, in the form of a gel, characterised in that it comprises:

   (a) from 10,000 to 1,000,000 units Nystatin per g of the composition;

   (b) from 5 to 20% by weight of hydrocolloid polymer having a molecular weight of from 10,000 to 20,000 derived from degraded carrageenan;

   (c) from 0.1 to 5% by weight of a thickening agent other than carrageenan as excipient; and

   (d) from 50 to 93% by weight of water.

7. A composition according to any of claims 1 to 5, in the form of a gel, characterised in that it comprises:

   (a) from 0.3 to 30% by weight of Nystatin having an activity of at least 3,000,000 U per g;

   (b) from 5 to 20% by weight of hydrocolloid polymer having a molecular weight of from 10,000 to 20,000 derived from degraded carrageenan;

   (c) from 0.1 to 5% by weight of a thickening agent other than carrageenan as excipient; and

   (d) from 50 to 93% by weight of water.

## Revendications

9

1. Composition pour application topique à la peau et à la muqueuse, notamment aux muqueuses orale et génitale, pour le traitement d'infections, comprenant:

   (a) une quantité efficace du point de vue pharmacologique comprise entre 0,3 et 3% en poids d'un antibiotique; et

   (b) en tant qu'agent de fixation de l'antibiotique, de 1 à 50% en poids d'un polymère hydrocolloïde comprenant un polygalactoside sulfaté et présentant un poids moléculaire compris entre 10 000 et 20 000, qui est susceptible de former un gel.·

2. Une composition selon la revendication 1, caractérisée en ce que l'antibiotique est choisi parmi la liste suivante:

   . nystatine
   . nitrate de miconazole
   . acide lauryl-oxypropyl-$\beta$-aminobutyrique
   . amphotéricine B
   . acide undécylénique
   . chloroquinaldol
   . nitrate d'éconazole
   . natamycine
   . cloprothiazole
   . clotrimazole
   . tolnaftate
   . lucensomycine
   . tétracycline
   . érythromycine.

3. Une composition selon la revendication 1 ou 2, caractérisée en ce que l'agent de fixation est choisi parmi les carragénane lambda dégradée, carragénane kappa dégradée, carragénane iota dégradée ou leurs mélanges, présentant chacune un poids moléculaire compris entre 10 000 et 20 000.

4. Une composition selon la revendication 1 ou 2, caractérisée en ce que l'agent de fixation dérive de l'algue rouge Chondrus crispus.

5. Une composition selon la revendication 1 ou 2, caractérisée en ce que l'agent de fixation dérive de l'algue rouge Eucheuma spinosum.

6. Une composition selon l'une quelconque des revendications précédentes, sous forme de gel, caractérisée en ce qu'elle comprend:

   (a) de 10 000 à 1 000 000 d'unités de nystatine par gramme de composition;

   (b) de 5 à 20% en poids de polymère hydrocolloïde présentant un poids moléculaire compris entre 10 000 et 20 000, dérivant de carragénane dégradée;

   (c) de 0,1 à 5% en poids d'un agent épaississant autre qu'un carragénane agissant en tant qu'excipient; et

   (d) de 50 à 93% en poids d'eau.

7. Une composition selon l'une quelconque des revendications 1 à 5, sous forme de gel, caractérisée en ce qu'elle comprend:

   (a) de 0,3 à 30% en poids de nystatine présentant une activité d'au moins 3 000 000 d'unités par gramme;

   (b) de 5 à 20% en poids de polymère hydrocolloïde présentant un poids moléculaire compris entre 10 000 et 20 000 dérivant de carragénane dégradée;

   (c) de 0,1 à 5% en poids d'un agent épaississant autre qu'un carragénane agissant en tant qu'excipient; et

   (d) de 50 à 93% en poids d'eau.

**Patentansprüche**

1. Präparat für topische Behandlung der Haut und mukös- oder Schleimhäute, insbesondere für Mund- und Genitalschleimhäute, zu Behandlung der Infektionen, das enthält:

a) eine pharmakologisch wirksame Menge von 0.3 bis 3 Gewichtsprozent von einem Antibiotikum, und

b) als Fixiermittel für das Antibiotikum, von 1 bis 50 Gewichtsprozent von einem sulfatierten Polygalactoside enthaltenden hydrokolloiden gelierfähigen Polymers mit einem von 10.000 bis 20.000 Molekülargewicht.

2. Ein Präparat gemäss Anspruch 1, dadurch gekennzeichnet, dass das Antibiotikum in der Gruppe, die:
Nystatin
Mikonazol Nitrat
Lauryl oxypropyl- $\beta$ -amino Buttersäure
Amphoterizin B
Undecylen Säure
Chlorquinaldol
Econazol Nitrat
Natamyzin
Cloprothiazol
Clotrimazol
Tolnaftat
Lucensomyzin
Tetracyclin
Erythromyzin
enthält, ausgewählt ist.

3. Ein Präparat nach Anspruch 1 oder 2, dadurch gekennzeichnet, dass das Fixiermittel in der Gruppe der degradierten Lambda-Karrageenan, degradierten Kappa-Karrageenan, degradierten Iota-Karrageenan, oder ihren Mischungen, die ein von 10.000 bis 20.000 Molekülargewicht aufweisen, gewählt wird.

4. Ein Präparat nach Anspruch 1 oder 2, dadurch gekennzeichnet, dass das Fixiermittel von der roten Chondrus crispus Alge entnommen wird.

5. Ein Präparat nach Anspruch 1 oder 2, dadurch gekennzeichnet, dass das Fixiermittel von der roten Eucheuma spinosum Alge entnommen wird.

6. Ein Präparat nach einem der vorstehenden Ansprüche, in Form eines Gels, dadurch gekennzeichnet, dass dieses enthält:
(a) von 10.000 bis 1.000.000 Nystatin-Einheiten pro Gramm des Präparats;
(b) von 5 bis 20 Gewichtsprozent von einem 10.000 bis 20.000 Molekülargewicht Hydrocolloid Polymer das von degradiertem Karrageenan gewonnen wird;
(c) als Arzneistoffträger von 0.1 bis 5 Gewichtsprozent Verdickungsmittel anders als Karrageenan; und
(d) von 50 bis 93 Gewichtsprozent Wasser.

7. Zusammensetzung nach einem der Ansprüche 1 bis 5, in Form von Gel, dadurch gekennzeichnet, dass diese enthält:
(a) von 0.3 bis 30 Gewichtsprozent Nystatin mit einer Aktivität von wenigstens 3.000.000 Einheiten pro Gramm;
(b) von 5 bis 20 Gewichtsprozent von einem 10.000 bis 20.000 Molekülargewicht Hydrocolloid Polymer das von degradiertem Karrageenan gewonnen wird;
(c) als Arzneistoffträger von 0.1 bis 5 Gewichtsprozent Verdickungsmittel anders als Karrageenan; und
(d) von 50 bis 93 Gewichtsprozent Wasser.